# FASCICULE DE BREVET EUROPEEN

(11) **EP 1 741 403 B1**
(45) Date de publication et mention de la délivrance du brevet: **16.09.2009**
(21) Numéro de dépôt: 06291093.0
(22) Date de dépôt: 03.07.2006
(51) Int. Cl.: A61B 19/02

(54) **Récipient, notamment pour instruments chirurgicaux**
Behälter, insbesondere für chirurgische Vorrichtungen
Container, in particular for surgical instruments

(30) Priorité: 04.07.2005 FR 0507080
(43) Date de publication de la demande: 10.01.2007
(73) Titulaire: De Uthemann, Cyril, 1253 Vandoeuvres (CH)
(72) Inventeur: Despres, Jean-Albert, 41300 Souesmes (FR)
(74) Mandataire: Thinat, Michel

(56) Documents cités:
- DE-A1- 3 733 524
- US-A- 5 629 498
- US-A1- 2002 108 875
- US-B1- 6 629 602

## Description

L'invention concerne un récipient, notamment pour instruments chirurgicaux.

Un récipient de ce type est déjà connu par le document DE 3733524. Ce récipient connu est réalisé sous forme d'un plateau comportant une partie de récipient inférieure et une partie de récipient supérieure formant couvercle. La partie inférieure comprend un insert susceptible d'être amoviblement placé dans la partie inférieure et une plaque de recouvrement de cette dernière.

Ce récipient connu présente l'inconvénient de se composer d'un nombre de pièces relativement importantes qui doivent chacune être nettoyées séparément après chaque utilisation.

L'invention a pour but de pallier cet inconvénient.
Pour atteindre ce but, le récipient selon l'invention comporte les caractéristiques qui sont énoncées dans la partie caractérisante de la revendication 1.

D'autres caractéristiques de l'invention sont invoquées dans des revendications dépendantes.

Grâce à cette configuration des récipients selon l'invention, ceux-ci, en plus d'être facilement manipulables et conformes aux exigences les plus poussées d'hygiène, ceux-ci sont utilisables dans le cadre d'un procédé ou une installation entièrement automatique de tri d'instruments, notamment chirurgicaux.

L'invention sera mieux comprise, et d'autres buts, caractéristiques, détails et avantages de celle-ci apparaîtront plus clairement au cours de la description explicative qui va suivre faite en référence aux dessins schématiques annexés donnés uniquement à titre d'exemple illustrant un mode de réalisation de l'invention et dans lesquels :
- la figure 1 est une vue schématique, en perspective, d'une installation, non revendiquée, de tri automatique d'instruments chirurgicaux;
- la figure 2 est une vue de dessus d'un plateau de support d'un instrument selon l'invention ;
- la figure 3 est une vue éclatée, d'un plateau de support, selon la figure 2 ; et
- les figures 4 et 5 illustrent une configuration avantageuse des plateaux selon l'invention et d'un étage de stockage.

Pour pouvoir apprécier les avantages et particularités des récipients selon l'invention, on les décrit ci-après dans leur utilisation dans le cadre d'un procédé et d'une installation non-revendiqués de tri automatique d'instruments chirurgicaux. Bien entendu, la description est uniquement donnée à titre d'exemple, mais, de façon générale, l'invention est utilisable à toute opération qui implique une sélection et un rangement d'instruments spécifique pour la mise en oeuvre de l'opération.

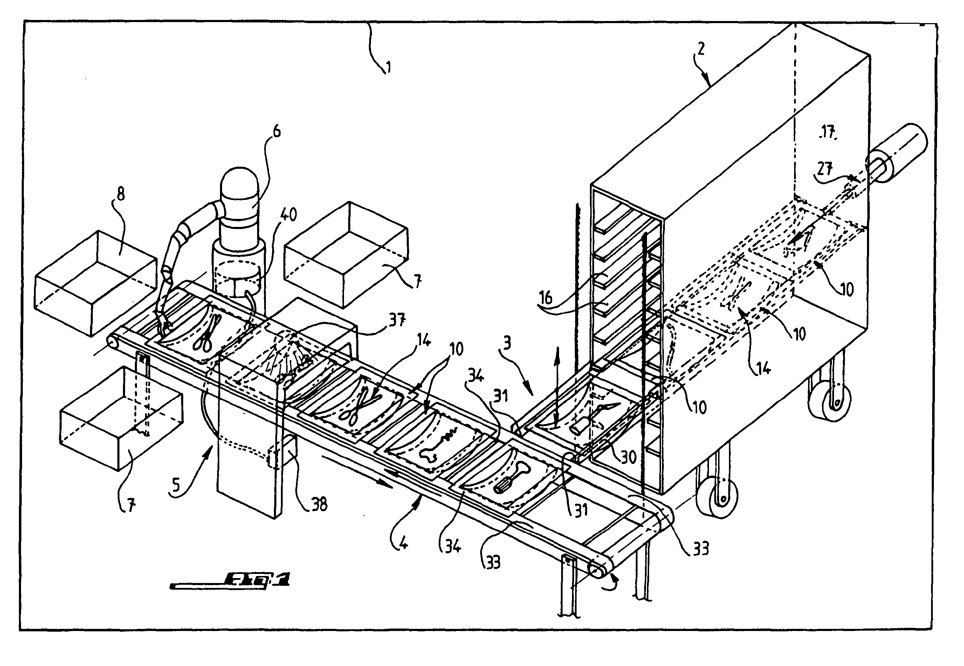

En se reportant à la figure 1, on constate qu'une installation, pour le tri des instruments nécessaires à une opération chirurgicale, comporte essentiellement, disposé à l'intérieur d'une enceinte à ambiance blanche 1, c'est-à-dire d'une propreté la plus parfaite possible, essentiellement un dispositif de stockage de l'ensemble des instruments chirurgicaux susceptibles d'être utilisés pour les différentes opérations chirurgicales possibles, à l'état propre, un dispositif 3 de transfert des instruments à un convoyeur 4 destiné à les transporter à un poste 5 de reconnaissance de la nature ou du type des instruments et un mécanisme de rangement des instruments après leur reconnaissance dans des récipients 7 dont chacun est destiné à contenir les instruments devant être utilisés pour une opération prédéterminée, un récipient 8 étant prévu pour la réception d'instruments jugés non conformes aux exigences établies pour les opérations chirurgicales.

Le dispositif 2 de stockage de l'ensemble des instruments est réalisé, dans le cas d'exemple, sous forme d'un chariot comportant un certain nombre de niveaux 9 de retenue chacun d'une pluralité de plateaux 10, dans l'exemple représenté 3, dont chacun peut comporter une pluralité d'alvéoles 12 de logement d'un instrument chirurgical 14. Dans le cas d'espèce, pour des raisons de simplification des dessins, chaque plateau ne comporte qu'une alvéole. Les plateaux 10 de chaque niveau 9 du chariot de stockage 2 sont supportés par des éléments de support en forme de rail de glissement 16 fixé chacun à une paroi latérale 17 du chariot orienté en direction du convoyeur 4.

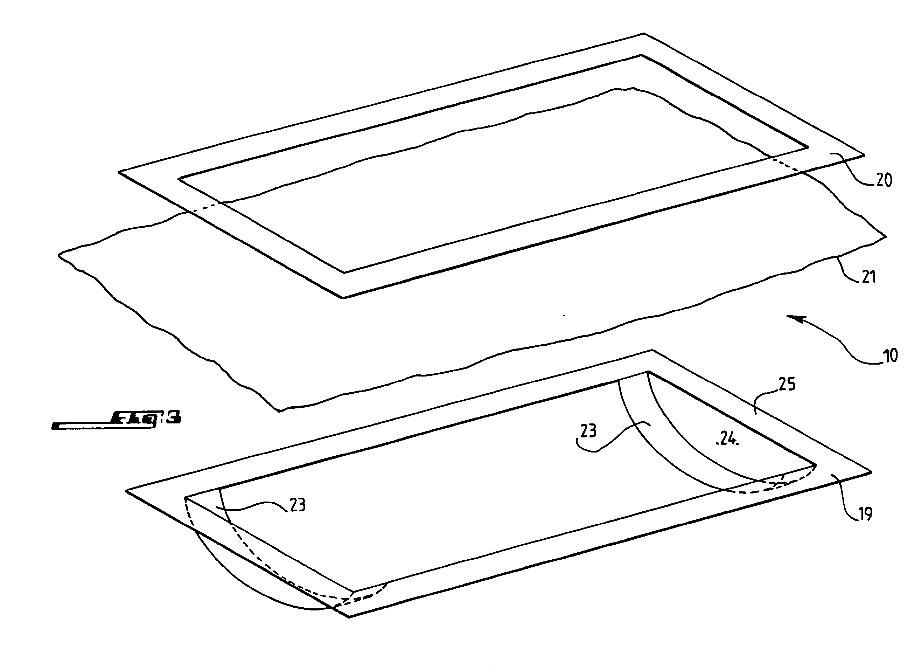

En se reportant à la figure 3, on constate que chaque plateau 10 se compose de deux cadres superposés, en un matériau facilement nettoyable, tel que de l'acier inoxydable, à savoir un élément de cadre inférieur 19 de forme générale rectangulaire et un cadre supérieur 20 de forme complémentaire et susceptible d'être fixé sur le cadre inférieur, par des moyens en forme de clips (non représentés), susceptibles de serrer le cadre supérieur sur le cadre inférieur pour qu'un élément 21 remplaçable, avantageusement à usage unique, en un matériau souple transparent aux rayons X, tel que du papier ou tissu, pourrait être inséré à deux de ses bords opposés entre les bords correspondants des deux cadres, pour former une alvéole 12 de logement d'un instrument 14 et ainsi le fond du plateau. Pour la formation aisée des alvéoles et leur maintien, le cadre inférieur 19 est pourvu d'éléments de support 23 en forme d'arcs, au niveau de chaque extrémité longitudinale. Il est avantageux que l'alvéole soit fermée à chaque extrémité longitudinale par une paroi verticale 24 formant l'espace entre le cadre et l'arc correspondant 23.

Comme on le voit sur la figure 1, chaque plateau 10 est déplaçable dans le chariot 2, perpendiculairement à son axe longitudinal en prenant appui par les côtés courts 25 sur les rails de glissement 16 du chariot.

L'agencement de transfert des plateaux 10 de chaque étage du chariot comporte, pour pousser les plateaux hors du chariot, un dispositif poussoir 27 et monté verticalement mobile à l'arrière du chariot 2, pour être positionnable à chaque niveau 9 du chariot. Le dispositif comporte un piston poussoir d'un vérin hydraulique qui, lors de son mouvement de sortie, pousse le dernier plateau et déplace ainsi l'ensemble de plateaux en direction du convoyeur.

Le dispositif de transfert 3 comporte en outre, à l'avant du chariot de stockage 2 de plateaux 10 un cadre 30 de transfert des plateaux 10 du chariot 2 au convoyeur 4, qui est verticalement déplaçable pour pouvoir recevoir les plateaux de chaque niveau 9 de stockage du chariot et pour les transporter ensuite à la hauteur du convoyeur 4 pour que les plateaux puissent être posés sur ce dernier. Plus précisément, dans l'exemple représenté, le cadre de transfert comporte essentiellement deux rails de coulissement 31, chacun étant susceptible d'être aligné, dans une position de réception de plateau d'un niveau ou étage 9, à un rail de glissement 16 de niveau, de façon que les plateaux 10 puissent être déplacés, sous l'effet du dispositif poussoir 27 des rails 16 du chariot aux rails 31 du cadre de transfert 30. Le cadre de transfert est dimensionné de façon à transférer successivement les plateaux 10 au convoyeur 4.

Le convoyeur 4 est montré, sur la figure 1, comme étant réalisé sous forme d'une bande de transport sans fin comportant, essentiellement deux brins plats parallèles 33, destinés au transport des plateaux 10, reliés par des traverses 34.

Pour assurer le transfert des plateaux 10 du cadre de transfert 30 aux brins de convoyeur 33, les rails de support 31 du cadre de transfert s'étendent jusqu'au-dessus du convoyeur et sa portion d'appui de plateau, dans sa position avant de transfert, est abaissable pour permettre la pose des chariots sur les brins du convoyeur situés en dessous. Puis ils s'écartent latéralement et reviennent dans leur position de réception d'un nouveau plateau.

Le convoyeur 4 transporte les plateaux reçus du chariot de stockage 2, par l'intermédiaire du cadre de transfert 3 au poste de reconnaissance 5 des instruments placés dans les alvéoles 12 des plateaux 10.

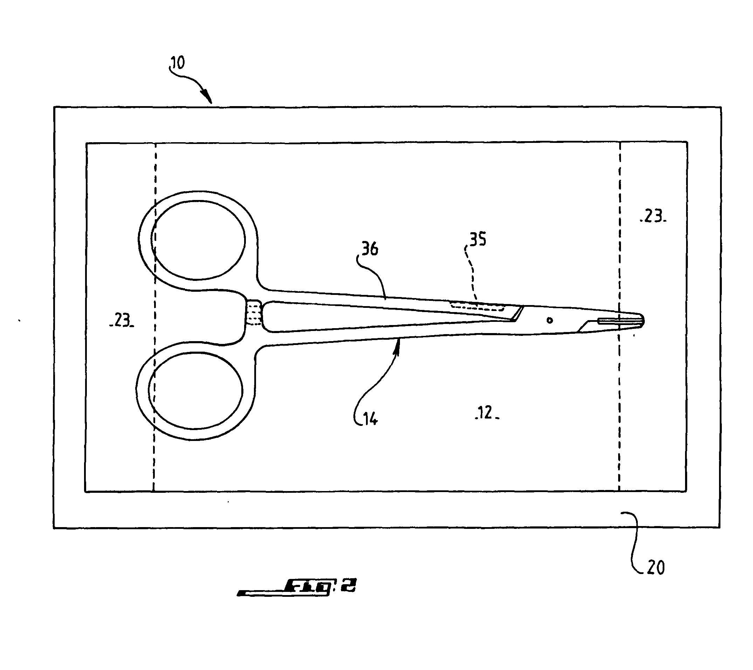

Concernant les instruments chirurgicaux, de fonctions et de formes différentes, ils sont chacun pourvus d'un code d'identité, reconnaissable par le poste 5 de façon que celui-ci soit en mesure de distinguer les instruments apportés par le convoyeur 4 selon leur type spécifique. Plus précisément, le code d'identité de chaque instrument est marqué, sous toute forme appropriée, sur un insert 35 qui est placé dans une cavité appropriée usinée dans l'instrument et fermée ensuite. La figure 2 montre à titre d'exemple, une pince chirurgicale dans une des branches notée 36 de laquelle est incorporé un insert 35.

Le poste de reconnaissance des instruments est avantageusement un lecteur aux rayons X, en forme d'un portique, à travers lequel passe le convoyeur 4 et qui comporte, disposée au-dessus du convoyeur 4, une source de rayons X 37, tandis qu'un récepteur des rayons ayant traversé l'instrument 14 est disposé sous le convoyeur.

Pour pouvoir reconnaître le type d'un instrument, d'après son insert, les éléments de celui-ci, qui constituent le code d'identification, doivent être moins transparents aux rayons X que le matériau constitutif de l'instrument. Ce code pourrait résider par exemple dans la forme de l'insert ou des marques prévues sur celui-ci ou encore la forme ou l'emplacement d'une encoche pratiquée dans l'insert. Des inserts de ce genre étant connus en soi, il n'est pas nécessaire de le décrire plus en détail. Les inserts sont avantageusement réalisés en un matériau relativement opaque aux rayons X. Ils pourraient être faits en laiton ou un alliage à base de laiton, alors que les instruments sont en acier inoxydable. De façon générale, pour le choix des matériaux, plus les atomes constitutifs du matériau sont lourds, c'est-à-dire d'une masse atomique élevée, plus ils absorbent les rayons X, donc plus le matériau est opaque à ces rayons X. Pour assurer une reconnaissance fiable des instruments, il est nécessaire que les instruments soient présentés au poste de reconnaissance dans une position toujours clairement définie.

Après la reconnaissance des instruments, par lecture de leurs inserts, à l'aide des rayons X, l'appareil de rangement 6 saisit les instruments et les range dans les récipients 7 en forme de boîte, sous les ordres d'un dispositif informatique 40.

Ce dispositif comporte, dans sa mémoire, des protocoles d'opération, un protocole pour chaque type d'opération, qui indique les instruments devant être utilisés au cours de l'opération, le cas échéant dans leur ordre d'utilisation. Etant donné qu'à chaque type d'opération correspond un boîtier 7, les instruments devant être rangés dans cette boîte sont indiqués par le protocole établi pour cette opération. A cette fin, le dispositif informatique 40 identifie tout d'abord, d'après le signal qu'il vient de recevoir du lecteur 38 du poste de reconnaissance, le type de l'instrument qui vient d'être examiné et détermine, en se reportant aux différents protocoles à quel type d'opération et ainsi à quelle boîte 7 un instrument de ce type est destiné. Puis, il donne l'ordre au dispositif de rangement 6 de saisir l'instrument identifié dans le plateau et de le ranger dans la boîte appropriée.

En comparant les instruments rangés dans une boîte à ceux figurant dans le protocole, le dispositif informatique connaît à tout moment l'état de "remplissage" de chaque boîte 7. S'il constate qu'une boîte est terminée, c'est-à-dire contient tous les instruments nécessaires pour l'opération considérée, la boîte est fermée, par exemple par la mise en place de son couvercle.

L'installation prévoit également la possibilité d'écarter des instruments jugés non aptes à être utilisés, du circuit d'utilisation, en les plaçant dans une boîte de rebus 8. Diverses raisons pourraient motiver cette mesure, par exemple l'usure d'un instrument, l'impossibilité de l'identifier ou parce qu'il s'agit d'un instrument souillé.

Concernant le poste de rangement 6, des appareils susceptibles de fonctionner de la manière décrite plus haut, sont largement connus, si bien qu'il n'est pas nécessaire de décrire l'appareil utilisé dans le cadre de l'invention, précisément. Il convient d'indiquer qu'un tel appareil comporte un bras robot capable de saisir les instruments dans leur plateau et de les placer ensuite, en fonction des instructions reçues du dispositif informatique, dans les boîtes appropriées.

Concernant le fonctionnement de l'installation et le déroulement du procédé ainsi que les différentes étapes de celui-ci, ils découlent de la description qui vient d'être faite. Il va sans le préciser davantage, que pour chaque transfert d'un plateau sur le convoyeur, celui-ci est arrêté pendant un bref instant de temps nécessaire à la pose du plateau. Les arrêts du convoyeur pour le chargement des plateaux et le processus de reconnaissance des instruments par le poste de reconnaissance ainsi que le rangement des instruments sont coordonnés par le dispositif informatique.

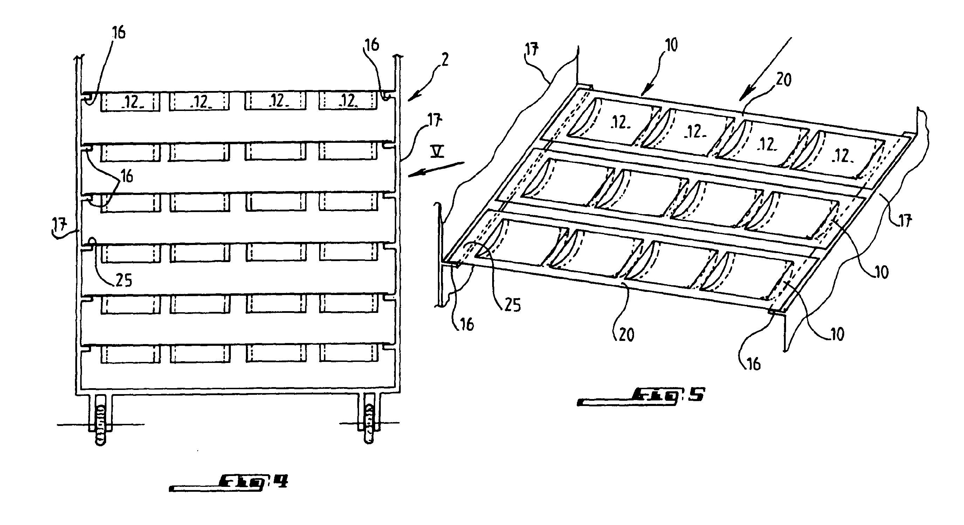

La description de l'installation, qui vient d'être faite, n'a été donnée qu'à titre d'exemple et des multiples modifications peuvent être apportées sans sortir du cadre de l'installation. Pour augmenter la capacité de stockage du chariot 2, chaque plateau 10 pourrait comporter quatre alvéoles, comme le montrent les figures 4 et 5. La flèche symbolise l'action du mécanisme de déplacement du plateau vers le convoyeur.3

## Revendications

1. Récipient, notamment pour contenir des instruments chirurgicaux, **caractérisé en ce qu'**il est réalisé sous forme d'un plateau (10) comprenant un cadre (19, 20) rigide et un fond remplaçable en un matériau (21) avantageusement à usage unique, tel que du papier ou du tissu.

2. Récipient selon la revendication 1, **caractéris,é en ce que** le cadre rigide est formé par superposition de deux cadres (19, 20) élémentaires enserrant entre eux les bords de l'élément de fond (21) de façon que celui-ci forme une alvéole (12) de logement d'un instrument (14), à l'intérieur du cadre.

3. Récipient selon l'une des revendications 1 ou 2, **caractérisé en ce que** les cadres élémentaires (19, 20) et l'élément de fond (21) sont de forme rectangulaire et l'élément de fond est inséré entre les deux cadres le long de deux côtés opposés.

4. Récipient selon l'une des revendications 2 ou 3, **caractérisé en ce que** le plateau (10) comporte à chaque extrémité libre d'une alvéole (12) un arc de support (23) du bord arqué de l'élément de fond en forme d'alvéole, qui est fixé au cadre inférieur (19).

5. Récipient selon la revendication 4, **caractérisé en ce que** l'espace entre l'élément de cadre inférieur (19) et l'arc de support (23) est fermé par une paroi (24) de fermeture latérale de l'alvéole (12).

6. Récipient selon l'une des revendications 2 à 5, **caractérisé en ce que** le plateau (10) comporte une pluralité d'alvéoles juxtaposées (12).

7. Récipient selon l'une des revendications 1 à 6, **caractérisé en ce que** les cadres (19, 20) sont réalisés en un matériau rigide facilement nettoyable, tel que de l'acier inoxydable.

## Claims

1. A container, in particular for containing surgical instruments, **characterized in that** it is made as a tray (10) comprising a rigid frame (19, 20) and an exchangeable bottom of a material (21), which is advantageously disposable, such as paper or fabric.

2. The container according to claim 1, **characterized in that** the rigid frame is formed by stacking two basic frames (19, 20) gripping therebetween the edges of the bottom element (21) so that the latter forms a compartment (12) for receiving an instrument (14) within the frame.

3. The container according to any of claims 1 or 2, **characterized in that** the basic frames (19, 20) and the bottom element (21) are of rectangular shape, and the bottom element is inserted between both frames along two opposite sides.

4. The container according to any of claims 2 or 3, **characterized in that** the tray (10) comprises at each free end of a compartment (12) a supporting curve (23) of the arcuate edge of the compartment-shaped bottom element, which is fastened to the lower frame (19).

5. The container according to claim 4, **characterized in that** the space between the lower frame element (19) and the supporting curve (23) is closed by a wall (24) for laterally closing the compartment (12).

6. The container according to any of claims 2 to 5, **characterized in that** the tray (10) comprises a plurality of adjacent compartments (12).

7. The container according to any of claims 1 to 6, **characterized in that** the frames (19, 20) are made of an easy to clean rigid material, such as stainless steel.

## Patentansprüche

1. Behälter, insbesondere um chirurgische Instrumente zu enthalten, **dadurch gekennzeichnet, dass** er in Form einer Schale (10) ausgebildet ist, die einen unbiegsamen Rahmen (19, 20) und einen austauschbaren Boden aus einem Material (21) umfasst, das vorteilhaft zum einmaligen Gebrauch gedacht ist, wie etwa Papier oder Stoff.

2. Behälter nach Anspruch 1, **dadurch gekennzeichnet, dass** der unbiegsame Rahmen durch das Überlagern von zwei Grundrahmen (19, 20) gebildet wird, die dazwischen die Ränder des Bodenelements (21) einklemmen, so dass dieses ein Fach (12) zur Aufnahme eines Instruments (14) im Innern des Rahmens bildet.

3. Behälter nach einem der Ansprüche 1 oder 2, **dadurch gekennzeichnet, dass** die Grundrahmen (19, 20) und das Bodenelement (21) rechteckig geformt sind und das Bodenelement zwischen den beiden Rahmen an zwei gegenüberliegenden Seiten entlang eingefügt wird.

4. Behälter nach einem der Ansprüche 2 oder 3, **dadurch gekennzeichnet, dass** die Schale (10) an jedem freien Ende eines Fachs (12) einen Stützbogen (23) des bogenförmigen Randes des fachförmigen Bodenelements umfasst, der an dem unteren Rahmen (19) befestigt ist.

5. Behälter nach Anspruch 4, **dadurch gekennzeichnet, dass** der Raum zwischen dem unteren Rahmenelement (19) und dem Stützbogen (23) von einer Wand (24) zum seitlichen Schießen des Fachs (12) gebildet wird.

6. Behälter nach einem der Ansprüche 2 bis 5, **dadurch gekennzeichnet, dass** die Schale (10) eine Vielzahl von nebeneinanderliegenden Fächern (12) umfasst.

7. Behälter nach einem der Ansprüche 1 bis 6, **dadurch gekennzeichnet, dass** die Rahmen (19, 20) aus einem leicht zu reinigenden unbiegsamen Material bestehen, wie etwa aus rostfreiem Stahl.
